(19) Europäisches Patentamt
European Patent Office
Office européen des brevets

(11) Veröffentlichungsnummer: **0 202 522**
**B1**

(12) **EUROPÄISCHE PATENTSCHRIFT**

(45) Veröffentlichungstag der Patentschrift: **13.09.89**

(51) Int. Cl.[4]: **C07B 57/00,** C07C 91/04

(21) Anmeldenummer: **86106089.5**

(22) Anmeldetag: **03.05.86**

(54) **Verfahren zur Racematspaltung von D,L-2-Aminobutan-1-ol.**

(30) Priorität: **11.05.85 DE 3517108**

(43) Veröffentlichungstag der Anmeldung: **26.11.86 Patentblatt 86/48**

(45) Bekanntmachung des Hinweises auf die Patenterteilung: **13.09.89 Patentblatt 89/37**

(84) Benannte Vertragsstaaten: **BE CH DE FR GB IT LI NL**

(56) Entgegenhaltungen:
**EP-A- 0 000 518**
**EP-A- 0 036 265**

(73) Patentinhaber: **BASF Aktiengesellschaft, Carl-Bosch-Strasse 38, D-6700 Ludwigshafen(DE)**

(72) Erfinder: **Lanzendoerfer, Franz, Dr., Bruchstrasse 40, D-6701 Ellerstadt(DE)**
Erfinder: **Fritz, Gerhard, Dr., Limburgerstrasse 23, D-6701 Dannstadt-Schauernheim(DE)**
Erfinder: **Siegel, Hardo, Dr., Hans-Purrman-Allee 25, D-6720 Speyer(DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die vorliegende Erfindung betrifft ein neues Verfahren zur Racematspaltung von D,L-2-Aminobutan-1-ol durch Umsetzung des Racemates mit einer optisch aktiven Carbonsäure, fraktionierte Kristallisation der hierbei entstehenden diastereomeren Salzpaare und Freisetzung der optisch aktiven Aminobutanole in üblicher Weise.

Dieses Verfahren ist, sieht man von der erfindungsgemäßen Ausgestaltung ab, an sich bekannt, wobei nach der Monographie "Optical Resolution Procedures for Chemical Compounds", Vol. I, von P. Newman, Optical Resolution Information Center, Manhattan College, New York, Seiten 36 - 39, diverse Säuren vorgeschlagen wurden.

Diese Säuren weisen jedoch verschiedene Nachteile auf, sei es, daß sie teuer sind, daß sich ihre diastereomeren Salze in ihrem Kristallisationsverhalten nicht hinreichend unterscheiden oder sei es, daß ihre Wiedergewinnung verfahrenstechnisch zu aufwendig ist. Lediglich die L-Weinsäure fand bisher breitere Anwendung (s. z.B. GB-PS 1 188 185), jedoch läßt auch die Weinsäure als Spaltreagens für technische Zwecke zu wünschen übrig, da sie sich wegen ihrer hohen Wasserlöslichkeit aus den wäßrigen Lösungen, die man bei der Freisetzung des Aminobutanols erhält, nur mit großem Aufwand zurückgewinnen läßt.

Der Erfindung lag daher die Aufgabe zugrunde, D,L-2-Aminobutan-1-ol auf einfachere und wirtschaftlichere Weise in seine optischen Antipoden zu spalten als bisher.

Demgemäß wurde ein neues Verfahren zur Racematspaltung von D,L-2-Amino-butan-1-ol durch Umsetzung des Racemats mit einer optisch aktiven Carbonsäure, fraktionierte Kristallisation der hierbei entstehenden diastereomeren Salzpaare und Freisetzung der optisch aktiven Aminobutanole gefunden, welches dadurch gekennzeichnet ist, daß man hierzu als optisch aktive Carbonsäure eine 2-Phenoxypropionsäure der allgemeinen Formel I

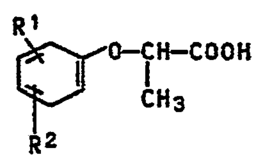

verwendet, in der $R^1$ eine $C_1$-$C_4$-Alkyl- oder Alkoxygruppe, die Hydroxylgruppe, Chlor oder die Trifluormethylgruppe bedeutet und $R^2$ für Wasserstoff oder $R^1$ steht, und daß man als Kristallisationsmedium ein organisches Lösungsmittel verwendet.

Es wurde weiterhin gefunden, daß sich die 2-(4-Chlor-2-methylphenoxy)-propionsäure und die 2-(2,4-Dichlorphenoxy)-propionsäure, jeweils in einer ihrer optisch aktiven Formen, für den erfindungsgemäßen Zweck besonders empfehlen.

Die optisch aktiven Säuren I sind größtenteils bekannt und lassen sich in guten Ausbeuten durch Umsetzung von entsprechend substituierten Phenolaten mit Alkalisalzen von optisch aktiven Chlorpropionsäuren herstellen. Einzelheiten über dieses Herstellungsverfahren sind der DE-PS 1 543 841 zu entnehmen.

Ein besonderer Vorteil des erfindungsgemäßen Verfahrens liegt darin, daß die Salze der jeweiligen diastereomeren Salzpaare in organischen Lösungsmitteln beträchtliche Löslichkeitsunterschiede aufweisen und daher eine bequeme Trennung in Kristallisat und Mutterlauge gestatten.

Als Lösungsmittel kommen vornehmlich polare Flüssigkeiten mit Siedepunkten zwischen 50 und 150°C in Betracht, also z.B. Aromaten wie Toluol und Chlorbenzol, Chloralkane wie Methylenchlorid und die Chlorethane, Ketone wie Aceton und Methylethylketon, Ether wie Dipropylether, Diisopropylether, Methyl-tert.-butylether und Tetrahydrofuran und Ester wie Methylacetat, Ethylacetat, Propylacetat, Isobutylacetat und Methylpropionat. Unter den Alkoholen eignen sich beispielsweise die Butanole, wogegen sich Methanol, Ethanol und die Propanole weniger empfehlen, da die Löslichkeitsunterschiede der Salze hierin im Bereich der Temperaturen um Raumtemperatur relativ gering sind. Im übrigen genügen einfache Vorversuche, um die Brauchbarkeit eines Lösungsmittels zu ermitteln. Das gleiche gilt für die Mengen der Lösungsmittel, die im allgemeinen zwischen 1 und 6 l pro kg des DL-Aminobutanols liegen.

Nach den bisherigen Beobachtungen sind die Salze aus den D-Säuren I mit dem D-Aminobutanol schwerer oder sogar wesentlich schwerer löslich als die diastereomeren D,L-Salze; das gleiche gilt natürlich für das Verhältnis der L,L-Salze zu den L,D-Salzen.

Methodisch kann man im übrigen wie üblich vorgehen. Beispielsweise kann man die Lösung des D,L-Aminobutanols vorlegen und diese mit der Lösung einer Säure I versetzen und anschließend erwärmen. In der Regel setzt man hierbei nicht mehr als 1 mol der Säure I pro Mol des Racemates ein. Häufig empfiehlt es sich auch, weniger Säure, etwa nur 0,5 mol/mol I zu verwenden, und zwar wenn eines der Salze besonders bevorzugt kristallisiert.

Die bei höherer Temperatur - meistens 40 - 70°C - homogene Salzlösung wird sodann unter mäßigem Rühren allmählich abgekühlt, wobei das schwerer lösliche Salz, gegebenenfalls nach Zusatz einiger Impfkristalle, auskristallisiert. Zur Erhöhung der optischen Reinheit kann man das abgetrennte Salz noch waschen oder auch ein- oder mehrmals wie üblich umkristallisieren.

Auch die Freisetzung des optisch aktiven Aminobutanols aus dem Salz kann wie üblich erfolgen, wobei das erfindungsgemäße Verfahren den verfahrenstechnisch besonderen Vorteil bietet, daß die Säuren I praktisch wasserunlöslich sind und daher ohne irgendwelchen Destillations- oder Extraktionsaufwand zurückgewonnen werden können.

Beispielsweise kann man das Salz oder dessen Lösung in einem wasserunlöslichen Lösungsmittel mit einer starken wäßrigen Mineralsäure wie Schwefelsäure oder Salzsäure versetzen, die Phasen trennen und das Aminobutanol mittels einer Mineralbase wie Natronlauge aus der wäßrigen Lösung des mineralsauren Salzes des Aminobutanols in

Freiheit setzen und wie üblich destillativ oder extraktiv - z.B. mit Isobutanol als Extraktionsmittel - in reiner Form gewinnen.

Ebensogut lassen sich die Teilschritte dieses Verfahrensganges auch vertauschen, d.h. man kann das diastereomere Salz auch zunächst mit einer Mineralbase zerlegen, das Aminobutanol abtrennen und die Säure I durch Ansäuern ihrer Salzlösung in Freiheit setzen und wieder zurückgewinnen. In beiden Fällen können die wäßrigen Phasen auch mittels Ionenaustauschern aufgearbeitet werden.

Die diastereomeren Salze aus der Säure I und den optisch aktiven 2-Amino-butan-1-olen sind neue Verbindungen und lassen sich häufig auch in dieser Form für weitere Umsetzungen verwenden.

Beispiele

In den folgenden Beispielen wurde jeweils der Drehwert des gewonnenen Aminobutanols ermittelt und aus diesem wie üblich die Ausbeute an dem überwiegend vorhandenen Enantiomeren bestimmt.

Beispiel 1

Eine auf 50°C erwärmte Lösung aus 470 g (2 mol) D-(+)-2-(2,4-Dichlorphenoxy)-propionsäure, 178 g (2 mol) D,L-2-Aminobutan-1-ol und 1000 ml Isobutylacetat wurde unter langsamen Rühren im Laufe von 5 Stunden auf 10°C abgekühlt und weitere 2 Stunden bei dieser Temperatur belassen, wobei 275 g (85 %) Salz auskristallisierten.

Das Salz wurde mit 416 g 30 gew.%iger wäßriger Schwefelsäure (= 1,27 mol Säure) versetzt, wobei sich die Spaltsäure als kristalline Masse abschied. Die wäßrige Phase wurde sodann mit 135 g 50 gew.%iger Natronlauge versetzt und mit 500 g Isobutanol extrahiert. Die Destillation des Extraktes lieferte das 2-Aminobutanol in einer Ausbeute von 94 %. Hiervon (= 100 %) entfielen 93 % auf das D-(+)-2-Aminobutan-1-ol und der Rest auf das antipodische Isomere. Die gleiche optische Reinheit hatte das diastereomere Salz; Smp. 111 - 112°C.

Beispiel 2

Ebenso wie in Beispiel 1, jedoch mit 700 ml Methyl-tert.-butylether als Lösungsmittel, wurde das D-(+)-2-Aminobutan-1-ol in einer Ausbeute von 93 % und einer optischen Reinheit von 95 % hergestellt.

Beispiel 3

Analog Beispiel 1, jedoch mit 1000 ml Ethylacetat als Lösungsmittel und Abkühlen auf 15°C wurde ein Kristallisat gewonnen, welches vor seiner Weiterverarbeitung noch 2 h bei 30°C mit 1200 ml frischem Ethylacetat gerührt wurde. Die Ausbeute an D-(+)-2-Aminobutan-1-ol betrug in diesem Falle 82 % und die optische Reinheit 98 %.

Beispiel 4

Eine auf 50°C erwärmte Lösung aus 215 g (1 mol) D-(+)-2-(4-Chlor-2-methylphenoxy)-propionsäure, 89 g (1 mol) D,L-2-Aminobutan-1-ol und 500 ml Ethylacetat wurde im Laufe von 20 h unter mäßigem Rühren auf 0°C abgekühlt und wie in Beispiel 1 weiterverarbeitet. Die Ausbeute an D-(+)-2-Aminobutan-1-ol betrug in diesem Falle 81 % und die optische Reinheit 94 %. Smp. des diastereomeren Salzes: 100 - 102°C.

Beispiel 5

Eine Salzlösung wie in Beispiel 3 wurde bei 50°C mit einigen Kristallen des zu gewinnenden diastereomeren Salzes versetzt, 1 h bei 50°C gehalten, im Laufe von weiteren 9 h auf 40°C, weiteren 8 h auf 30°C und schließlich innerhalb von weiteren 7 h auf 5°C gekühlt. Die Aufarbeitung des Kristallisates lieferte das D-(+)-2-Aminobutan-1-ol in einer Ausbeute von 88 % und einer optischen Reinheit von 98 %.

Beispiel 6

Analog Beispiel 1, jedoch mit 500 ml Isobutanol und eine Abkühlung auf 5°C wurde das D-(+)-2-Aminobutan-1-ol in 90 %iger Ausbeute und 95 %iger optischer Reinheit hergestellt.

Beispiel 7

Analog Beispiel 6, jedoch mit D-(+)-2-(4-Chlor-2-methylphenoxy)-propionsäure und 600 ml Isobutanol wurde das D-(+)-2-Aminobutanol in 87 %iger Ausbeute und 94 %iger optischer Reinheit gewonnen.

Beispiel 8

Analog Beispiel 1, jedoch mit 1800 ml Toluol als Lösungsmittel wurde das D-(+)-2-Aminobutan-1-ol in einer Ausbeute von 90 % und einer optischen Reinheit von 99 % gewonnen.

**Patentansprüche**

1. Verfahren zur Racematspaltung von D,L-2-Aminobutan-1-ol durch Umsetzung des Racemats mit einer optisch aktiven Carbonsäure, fraktionierte Kristallisation der hierbei entstehenden diastereomeren Salzpaare und Freisetzung der optisch aktiven Aminobutanole, dadurch gekennzeichnet, daß man hierzu als optisch aktive Carbonsäure eine 2-Phenoxypropionsäure der allgemeinen Formel I

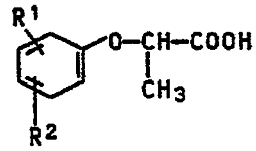

verwendet, in der $R^1$ eine $C_1$-$C_4$-Alkyl- oder Alkoxygruppe, die Hydroxylgruppe, Chlor oder die

Trifluormethylgruppe bedeutet und $R^2$ für Wasserstoff oder $R^1$ steht, und daß man als Kristallisationsmedium ein organisches Lösungsmittel verwendet.

2. Verfahren nach Anspruch 1, dadurch gekennzeichnet, daß man als Säure I eine optisch aktive 2-(2,4-Dichlorphenoxy)-propionsäure oder eine optisch aktive 2-(4-Chlor-2-methylphenoxy)-propionsäure verwendet.

3. Diastereomere Salze aus den optisch aktiven Säuren I einerseits und D- bzw. L-2-Aminobutan-1-ol andererseits.

4. Salz aus der D-2-(2,4-Dichlorphenoxy)-propionsäure und D-2-Aminobutanol.

5. Salz aus der D-2-(4-Chlor-2-methylphenoxy)-propionsäure und D-2-Aminobutanol.

## Claims

1. A process for resolving a racemate of D,L-2-aminobutan-1-ol by reaction of the racemate with an optically active carboxylic acid, fractional crystallization of the resulting diastereoisomeric salt pairs and liberation of the optically active aminobutanols, which comprises using for this purpose as the optically active carboxylic acid a 2-phenoxypropionic acid of the general formula I

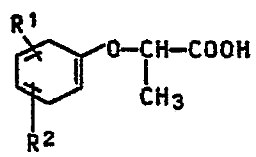

where $R^1$ is $C_1$-$C_4$-alkyl or -alkoxy, hydroxyl, chlorine or trifluoromethyl and $R^2$ is hydrogen or $R^1$, and an organic solvent as the crystallization medium.

2. A process as claimed in claim 1, wherein acid I is an optically active 2-(2,4-dichlorophenoxy)-propionic acid or an optically active 2-(4-chloro-2-methylphenoxy)-propionic acid.

3. A diastereoisomeric salt from an optically active acid I on the one hand and D- or L-2-aminobutan-1-ol on the other.

4. A salt from D-2-(2,4-dichlorophenoxy)propionic acid and D-2-aminobutanol.

5. A salt from D-2-(4-chloro-2-methylphenoxy)-propionic acid and D-2-aminobutanol.

## Revendications

1. Procédé de résolution optique du racémate D, L-2-aminobutane-1-ol par réaction du racémate avec un acide carboxylique optiquement actif, cristallisation fractionnée de la paire de sels diastéréoisomères ainsi formés et libération des aminobutanols optiquement actifs, caractérisé en ce que l'on utilise à cet effet, comme acide carboxylique optiquement actif, un acide 2-phénoxypropionique de formule générale I

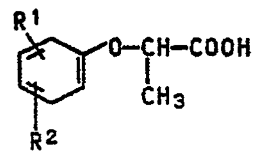

dans laquelle $R^1$ désigne un groupe alkyle ou alkoxy en $C_1$–$C_4$, le groupe hydroxyle, un atome de chlore ou le groupe trifluorométhyle et $R^2$ représente de l'hydrogène ou $R^1$, et en ce que l'on utilise comme milieu de cristallisation un solvant organique.

2. Procédé suivant la revendication 1, caractérisé en ce que l'on utilise, comme acide I, un acide 2-(2,4-dichlorophénoxy)-propionique optiquement actif ou un acide 2-(4-chloro-2-méthylphénoxy)-propionique optiquement actif.

3. Sels diastéréoisomères des acides optiquement actifs I d'une part et du D- ou L-2-aminobutane-1-ol d'autre part.

4. Sel de l'acide D-2-(2,4-dichlorophénoxy)-propionique et du D-2-aminobutanol.

5. Sel de l'acide D-2-(4-chloro-2-méthylphénoxy)-propionique et du D-2-aminobutanol.